(19) Europäisches Patentamt
European Patent Office
Office européen des brevets

(11) Publication number: **0 063 027**
**B1**

(12) **EUROPEAN PATENT SPECIFICATION**

(45) Date of publication of patent specification: **09.07.86**

(51) Int. Cl.$^4$: **C 07 D 501/46**

(21) Application number: **82301831.2**

(22) Date of filing: **07.04.82**

(54) **Process for the preparation of imidazoledicarboxylic acid derivatives of cephalosporins.**

(30) Priority: **10.04.81 JP 53919/81**

(43) Date of publication of application:
**20.10.82 Bulletin 82/42**

(45) Publication of the grant of the patent:
**09.07.86 Bulletin 86/28**

(84) Designated Contracting States:
**CH DE FR GB LI**

(56) References cited:
**EP-A-0 019 067**
**EP-A-0 026 811**
**FR-A-2 394 550**
**JP-A-55 076 887**

(73) Proprietor: **AJINOMOTO CO., INC.**
**5-8, Kyobashi 1-chome, Chuo-ku**
**Tokyo 104 (JP)**

(72) Inventor: **Nakanishi, Eiji**
**No. 2-20-8 Kannon Kawasaki-ku**
**Kawasaki-shi Kanagawa-ken (JP)**
Inventor: **Iwagami, Hisao**
**No. 2-20-8, Kannon Kawasaki-ku**
**Kawasaki-shi Kanagawa-ken (JP)**
Inventor: **Yasuda, Naohiko**
**No. 2-36-2, Hairand**
**Yokosuka-shi Kanagawa-ken (JP)**

(74) Representative: **Jones, Michael Raymond et al**
**HASELTINE LAKE & CO. Hazlitt House**
**28 Southampton Buildings Chancery Lane**
**London WC2A 1AT (GB)**

**EP 0 063 027 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for the preparation of imidazole-dicarboxylic acid derivatives which can be used as antibiotic substances of infectious diseases caused by *Pseudomonas aeruginosa* in men and animals.

According to the present invention, there is provided a process for the preparation of an imidazole-dicarboxylic acid derivative represented by the following general formula:

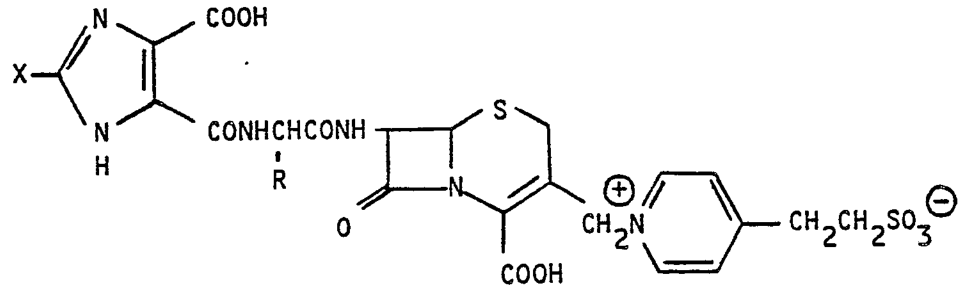

(I)

or an alkali metal salt thereof, wherein X is hydrogen, halogen, hydroxy, alkoxy, aralkoxy, aryloxy, mercapto, alkylthio, aralkylthio, arylthio, alkylsulfonyl aralkylsulfonyl, arylsulfonyl alkylsulfinyl, aralkylsulfinyl, arylsulfinyl, amino, mono- or di-alkylamino, mono- or di-aralkylamino, mono- or di-arylamino, acylamino, sulfonic acid, nitro, alkyl, aralkyl, aryl or heterocyclyl, and R is a hydrogen, alkyl, aralkyl, aryl, 4-hydroxyphenyl, or heterocyclyl, which comprises reacting a compound represented by the following general formula:

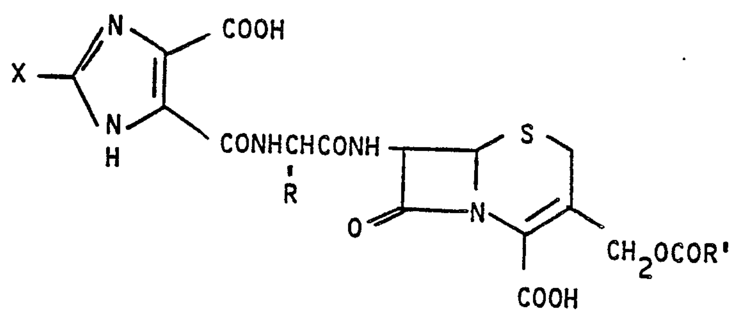

(II)

or an alkali metal salt thereof, wherein R and X are as defined above and R′ is alkyl, with 4-pyridineethanesulfonic acid or an alkali metal salt thereof, in formamide and in the presence of sodium iodide, potassium iodide or potassium thio-cyanate as a catalyst.

It has been found that the imidazole-dicarboxylic acid derivatives represented by the above general formula I have an antibiotic activity especially an antibiotic activity to *Pseudomonas aeruginosa*, and can be used as antibiotic substances (see Japanese Patent Application Laid-Open Specification No. 76887/80).

The amino acid constituting the imidazole-di-carboxylic acid derivatives prepared by the present invention, that is, the R-containing amino acid in the above general formula I, is, for example, phenylglycine or 4-hydroxyphenylglycine, and any of the L-, D- and DL-isomers may be used. From the viewpoint of antibiotic acid D-isomers are often preferred.

In the above general formula II, R′ is an alkyl group such as a methyl, ethyl, propyl or butyl group.

The 4-pyridineethanesulfonic acid used in the invention has the formula:

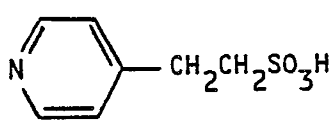

It is known that the introduction of the pyridinium group as in the above reaction is often accomplished by carrying out the reaction in an aqueous solution in the presence of potassium iodide, potassium thiocyanate or sodium iodide (see, for example, Japanese Patent Laid-Open Specification No. 76887/80).

In the case of such a reaction, conducted in an aqueous solution, decomposition of the starting compound occurs and therefore the yield is low.

We have made researches with a view to eliminating this defect and have found that when the above reaction is carried out in formamide, the intended compound can be obtained in a high yield. We have now completed the present invention based on this finding.

According to the present invention, using formamide as the reaction solvent, there can be attained a higher yield than the yield attainable by the conventional process in which the reaction is carried out in an

aqueous solution. Furthermore, the present invention is advantageous in that the amount of the sodium iodide, potassium iodide or potassium thiocyanate used as the catalyst can be reduced.

The reaction is preferably accomplished by adding 4-pyridineethanesulfonic acid to a solution obtained by dissolving an isolated compound of the general formula II in formamide, or to a formamide solution of a compound of the general formula II synthesized in formamide according to the process disclosed in Japanese Patent Application Laid-Open Specification No. 129287/80, adding a necessary amount of sodium iodide, potassium iodide or potassium thiocyanate as a catalyst, and, if desired, a base, and heating the mixture with stirring.

When the reaction is carried out by using alkali metal salts of the compound of the above general formula and 4-pyridineethanesulfonic acid, a base need not be added.

As the base for the reaction, there are preferably used tertiary amines such as triethylamine and diazobicyclononene and inorganic bases such as sodium hydrogencarbonate, sodium carbonate and potassium carbonate. Preferred reaction conditions are as follows: the amount of the base used is 1 to 5 equivalents, the amount of the catalyst used is 10 to 40 equivalents, the amount of 4-pyridineethanesulfonic acid used is 1 to 4 equivalents, each per equivalent of the compound of formula (II), the reaction temperature is 65 to 95°C, and the reaction time is 1 to 5 hours.

Isolation of the intended compound from the reaction liquid is preferably accomplished according to the following method. The reaction liquid is added to a great excess of a hydrophilic organic solvent capable of dissolving therein the sodium iodide, potassium iodide or potassium thiocyanate used as the catalyst to obtain the intended compound in solid form, dissolving the solid in water, adding the solution to the above organic solvent and repeating such operations several times, whereby the intended compound having a high purity can be obtained. As the organic solvent, there are preferably used acetone, methanol, ethanol, isopropanol, butanol, tetrahydrofuran, acetonitrile and mixtures of two or more of them. The solid obtained may be purified by various chromatographic techniques such as adsorption chromatography. In this case, water or a mixture of water with a hydrophilic organic solvent such as mentioned above may be used as the eluant.

The present invention will now be illustrated by the following Examples.

Example 1

In 40 ml of formamide, there were dissolved 5.87 g (10 millimoles) of disodium 7β-[D(−)-α-(4-carboxyimidazole-5-carboxyamido)-α-phenylacetamido]-3-acetoxymethyl-3-cephem-4-carboxylate and sodium 4-pyridineethanesulfonate, and 25 g of sodium iodide were added to the solution. Reaction was carried out at 80°C with stirring for 2.5 hours. The reaction liquid was naturally cooled and ice-cooled, and 300 ml of acetone were added dropwise to the reaction liquid with stirring. After the mixture had been cooled overnight, the solid formed was recovered by filtration and dissolved in 50 ml of water, and 300 ml of acetonee were added dropwise to the solution under ice-cooling and agitation to precipitate a solid. The solid formed was recovered by decantation and dissolved in 30 ml of water. The pH value of the solution was adjusted to 4, and 300 ml of ethanol were added dropwise to the solution under ice cooling and agitation to precipitate a solid. The liquid was cooled overnight and the solid formed was recovered by filtration and dried to obtain 5.6 g of the intended compound.

The solid obtained was dissolved in 30 ml of water, and the pH value was adjusted to 4. The solid was adsorbed on 100 ml of an adsorbent ("Amberlite XAD—7" supplied by Rohm and Haas Co.) packed in a column and the intended compound was eluted out with water. Fractions containing the intended compound were combined and concentrated until the volume was reduced to 30 ml. The pH value of the liquid was adjusted to 4, and 180 ml of ethanol were added dropwise to the liquid under ice cooling and agitation to precipitate a solid. The liquid was cooled overnight, and the solid formed was recovered by filtration and dried to obtain 2.5 g (the yield being 35.1%) of 7β-[D(−)-α-(4-carboxyimidazole-5-carboxyamido)-α-phenylacetamido]-3-(4-β-sulfoethylpyridinium)-methyl-3-cephem-4-carboxylic acid · 1.5 sodium salt · 0.5 hydrate.

Example 2

In 40 ml of formamide, there were dissolved 44.05 g (10 millimoles) of cephaloglycine, and 2.1 g of sodium hydrogencarbonate were added to the solution under ice cooling and agitation. Then, 3.6 g of a dihydrate of 5,10-dioxo-5,10-dihydroimidazo [1,5a,1',5'd] pyrazine-1,6-dicarboxylic acid were added little by little to the mixture. Reaction was carried out under ice cooling overnight. Insoluble substances were removed by centrifugal separation, and 0.56 g of sodium hydrogencarbonate, 4.4 g of sodium pyridine-ethanesulfonate and 38 g of sodium iodide were added to the supernatant with stirring. Reaction was carried out at 80°C with stirring for 2.5 hours. The intended product was separated and purified in the same manner as described in Example 1 to obtain 2.4 g (the yield being 33.7%) of 7β-[D(−)-(4-carboxyimidazole-5-carboxyamido)-α-phenylacetamido]-3-(4-β-sulfoethylpyridium)methyl-3-cephem-4-carboxylic acid 1.5 Na salt · 0.5 hydrate.

Referential Example 1

In 25 ml of water were suspended 5.43 g (10 millimoles) of 7β[D(−)-α-(4-carboxyimidazole-5-carboxyamido)-α-phenylacetamido]-cephalosporanic acid and 3.7 g (20 millimoles) of 4-pyridineethanesulfonic

acid, and the pH value of the suspension was adjusted to 6.5 by a 2N aqueous solution of sodium hydroxide to dissolve both the components. Then, 70 g of sodium iodide was added to the solution and reaction was carried out at 65°C for 70 minutes. The reaction liquid was post-treated in the same manner as described in Example 1 to obtain 2.0 g (the yield being 28.1%) of the intended compound (1.5 Na salt, 0.5 hydrate).

**Claims**

1. A process for the preparation of an imidazoledicarboxylic acid derivative represented by the following general formula:

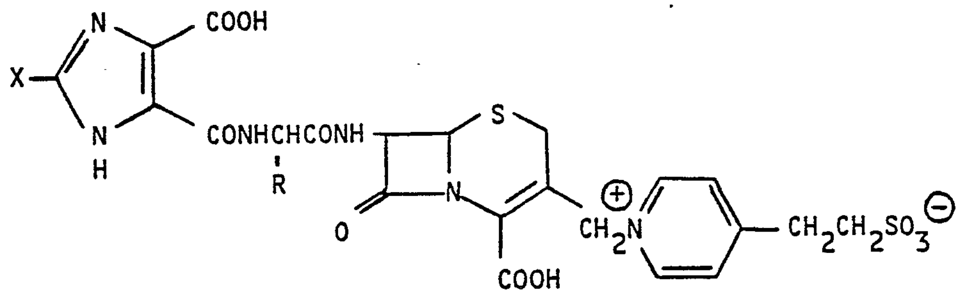

or an alkali metal salt thereof, wherein X is hydrogen, halogen, hydroxy, alkoxy, aralkoxy, aryloxy, mercapto, alkylthio, aralkylthio, arylthio, alkylsulfonyl aralkylsulfonyl, arylsulfonyl alkylsulfinyl, aralkylsulfinyl, arylsulfinyl, amino, mono- or di-alkylamino, mono- or di-aralkylamino, mono- or di-arylamino, acylamino, sulfonic acid, nitro, alkyl, aralkyl, aryl or heterocyclyl, and R is a hydrogen, alkyl, aralkyl, aryl, 4-hydroxyphenyl, or heterocyclyl, which comprises reacting a compound represented by the following general formula:

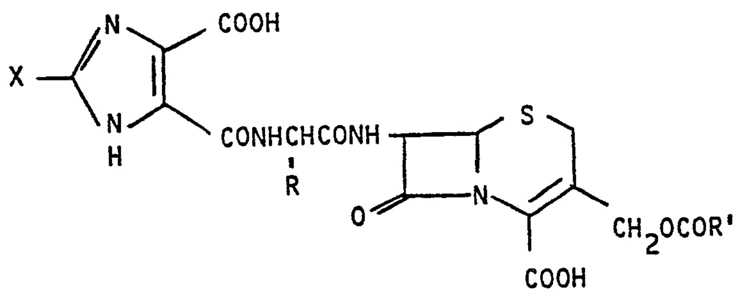

or an alkali metal salt thereof, wherein R and X are as defined above and R′ is alkyl, with 4-pyridineethanesulfonic acid or an alkali metal salt thereof, in formamide and in the presence of sodium iodide, potassium iodide or potassium thio-cyanate as a catalyst.

2. A process as claimed in claim 1, wherein R′ is methyl.

**Patentansprüche**

1. Verfahren zur Herstellung eines Imidazoldicarbonsäure-Derivats der folgenden allgemeinen Formel

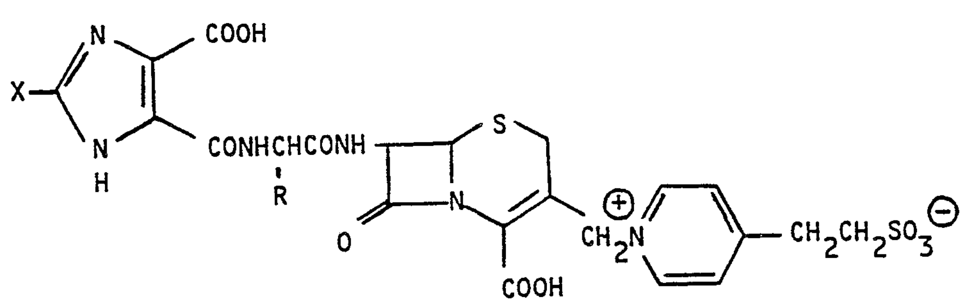

oder dessen Alkalimetallsalzen, worin X Wasserstoff, Halogen, Hydroxy, Alkoxy, Aralkoxy, Aryloxy, Mercapto, Alkylthio, Aralkylthio, Arylthio, Alkylsulfonyl, Aralkylsulfonyl, Arylsulfonyl, Alkylsulfinyl, Aralkylsulfinyl, Arylsulfinyl, Amino, Mono- oder Dialkylamino, Mono- oder Diaralkylamino, Mono- oder Diarylamino, Acylamino, eine Sulfonsäure-, Nitrogruppe, Alkyl, Aralkyl, Aryl oder eine heterocyclische

Gruppe (Heterocyclyl) bedeutet und R für Wasserstoff, Alkyl, Aralkyl, Aryl, 4-Hydroxyphenyl oder eine heterocyclische Gruppe (Heterocyclyl) steht, dadurch gekennzeichnet, daß eine Verbindung der allgemeinen Formel

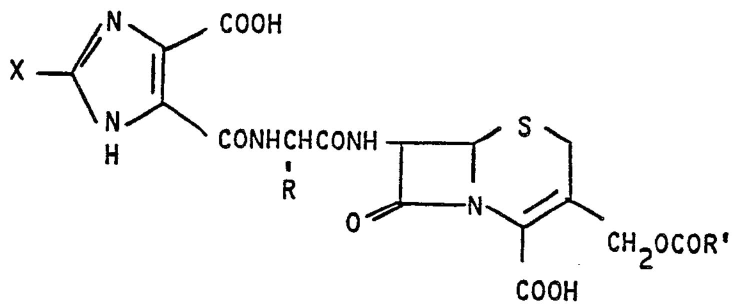

oder deren Alkalimetallsalz, wobei R und X die vorstehend gegebene Bedeutung haben und R′ Alkyl ist, mit 4-Pyridinethansulfonsäure oder deren Alkalimetallsalz in Gegenwart von Natriumiodid, Kaliumiodid oder Kaliumthiocyanat als Katalysator in Formamid umgesetzt wird.

2. Verfahren nach Anspruch 1, wobei R′ für Methyl steht.

**Revendications**

1. Procédé pour la préparation d'un dérivé d'acide imidazole-dicarboxylique représenté par la formule générale suivante:

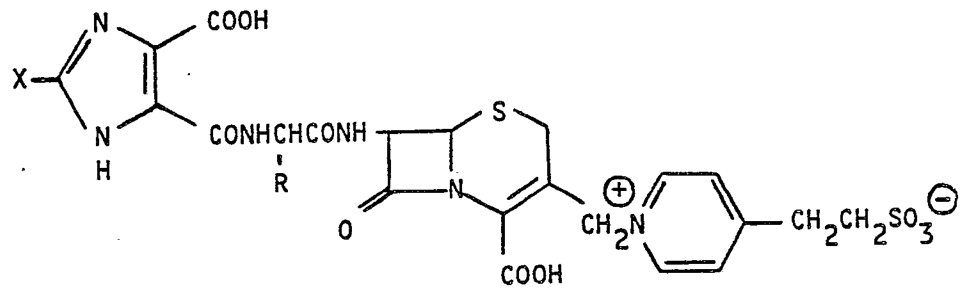

ou un sel de métal alcalin de ce dernier, dans laquelle X est un hydrogène, halogène, hydroxy, alcoxy, arylalcoxy, aryloxy, mercapto, alkylthio, arylalkylthio, arylthio, alkylsulfonyle, arylalkylsulfonyle, arylsulfonyle, alkylsulfinyle, arylalkylesulfinyle, arylsulfinyle, amino, mono- ou di-alkylamino, mono- ou di-arylalkylamino, mono- ou di-arylamino, acylamino, acide sulfonique, nitro, alkyle, arylalkyle, aryle ou hetérocyclyle et R est un hydrogène, alkyle, arylalkyle, aryle, 4-hydroxyphényle ou hétérocyclyle, qui comprend la réaction d'un composé représenté par la formule générale suivante:

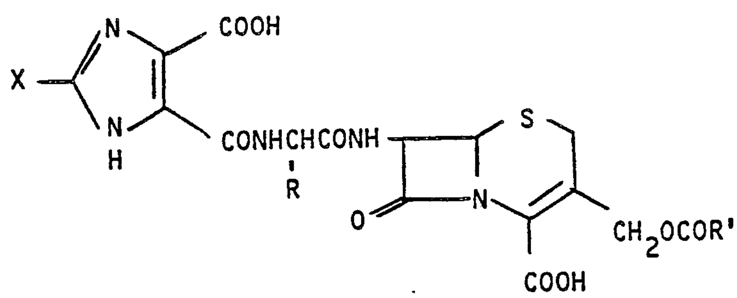

ou d'un sel de métal alcalin de ce dernier, dans laquelle R et X sont come définis précédemment et R′ est un alkyle, avec l'acide 4-pyridineéthanesulfonique ou un sel de métal alcalin de ce dernier, dans le formamide et en présence d'iodure de sodium, d'iodure de potassium ou de thiocyanate de potassium comme catalyseur.

2. Procédé selon la revendication 1, dans lequel R′ est un méthyle.